Europäisches Patentamt

European Patent Office

Office européen des brevets

(11) Publication number: **0 113 142**
**A2**

# EUROPEAN PATENT APPLICATION

(21) Application number: **83201717.2**

(22) Date of filing: **05.12.83**

(51) Int. Cl.³: **A 61 B 17/10**

(30) Priority: **06.12.82 IT 5403482 U**
**30.05.83 IT 5339483 U**
**01.06.83 IT 6760483**

(43) Date of publication of application:
**11.07.84 Bulletin 84/28**

(84) Designated Contracting States:
**AT BE CH DE FR GB LI LU NL SE**

(71) Applicant: **Ferrando, Ugo**
**C.so F. Turati 7**
**I-10128 Torino(IT)**

(71) Applicant: **Gardi, Giovanni**
**Via Baltimora 138**
**I-10137 Torino(IT)**

(71) Applicant: **Pagliano, Giustino**
**C.so Unione Sovietica 229**
**I-10134 Torino(IT)**

(72) Inventor: **Ferrando, Ugo**
**C.so F. Turati 7**
**I-10128 Torino(IT)**

(72) Inventor: **Gardi, Giovanni**
**Via Baltimora 138**
**I-10137 Torino(IT)**

(72) Inventor: **Pagliano, Giustino**
**C.so Unione Sovietica 229**
**I-10134 Torino(IT)**

(74) Representative: **Lotti, Giorgio**
**c/o Ing. Barzanò & Zanardo Milano S.p.A. Via Cernaia 20**
**I-10122 Torino(IT)**

(54) **Medical - surgical instrument.**

(57) A medical-surgical instrument is structurally and substantially constituted by forceps which, at the ends of their respective prongs, incorporate adjacent means for effecting the suture, respectively and simultaneously upstream and downstream of the relevant sector of a vessel or tissue in which a gradual operation involving incision and cutting is to be carried out by further means incorporated in said forceps, and performed by the surgeon.

Fig.1

EP 0 113 142 A2

## DESCRIPTION

This invention relates to a medical-surgical instrument for the clamping, obliteration and cutting of vessels and tissues, in particular under gradual conditions in a temporary or definitive manner, simultaneously upstream or downstream of the sector concerned, the cut being partial or complete.

Said surgical operations are conventionally carried out by means of an instrument of which the parts operate simultaneously in order to provide efficiency and operational simplicity, to the advantage of surgical rapidity.

The main object of the invention is to make considerable improvements to the known art by modifications to its components which can be easily and rapidly made, which allow them to be immediately assembled, and which result in prolonged operation with time, the overall result also being an economical advantage, ie reduced and thus commercially competitive costs.

A further object of the invention is to improve the functionality of the expulsion step of the suture clips, and to improve the manoeuvrability of the forceps, ie the carrying out of the clamping, by providing the operator with optimum vision of the location of said clips, these latter being, according to the invention, able to be inserted in the most varied positional directions, in addition to being suitable for use on tissues of organs having a surface which is not only flat but also curved, as for example in the case of the kidney, the bladder etc.

A further object is to attain the aforesaid by the use of means of simple and rapid construction, to provide an instrument of valid and reliable effectiveness, and with the further advantage of economy, ie reduced and thus competitive costs.

A further object of the invention is to obviate certain drawbacks

encountered in currently used instruments, such as the edge roughness of the cut, and the slowness of the operations themselves, these drawbacks resulting in the need for surgical ability in order to reduce these negative effects. To attain this end, the invention uses a new forceps configuration which makes them suitable for incorporating the means according to the invention, these means being arranged to advantageously determine rapid and clean excision with a certain degree of automation, in particular by means of a component which replaces the conventional metal bistoury, together with easy and reliable operation, simple construction with reduced overall size, the guarantee of durable operation with time, and thus resultant economy.

These objects and advantages together with others which will be apparent from the detailed description given hereinafter are attained by a medical-surgical instrument characterised by being structurally and substantially constituted by forceps which, at the ends of their respective prongs, incorporate adjacent means for effecting the suture respectively and simultaneously upstream and downstream of the relevant sector of a vessel or tissue in which a gradual operation involving incision and cutting is to be carried out by further means incorporated in said forceps, and performed by the surgeon.

A description is given hereinafter by way of non-limiting example of three embodiments of the invention with reference to the accompanying drawings in which:

Figure 1 is a complete frontal view of a first embodiment of the instrument according to the invention;

Figure 2 is an exploded perspective view similar to the preceding but to a larger scale, and showing the front sector of the instrument;

Figure 3 is an axial frontal view of a prong of the forceps of Figure 1;

Figure 4 shows a frontal and corresponding plan view respectively of an operating component;

Figure 5 is a partial plan view of the bistoury to a larger scale, taken along the arrow A of Figure 2;

Figure 6 is a frontal exploded detailed view to a larger scale of the front sector of a second embodiment of the forceps, effected in accordance with the principles of the invention;

Figure 7 is a corresponding complete view of Figure 6;

Figure 8 is a partial plan view of Figure 7, limited to the prong shown in the lower position;

Figure 9 is a complete view of a third embodiment of the surgical forceps according to the invention;

Figure 10 is a detail of the end of the forceps of Figure 9 to a larger scale;

Figure 11 is a constructional and functional modification, but graphically similar to the preceding Figure 10.

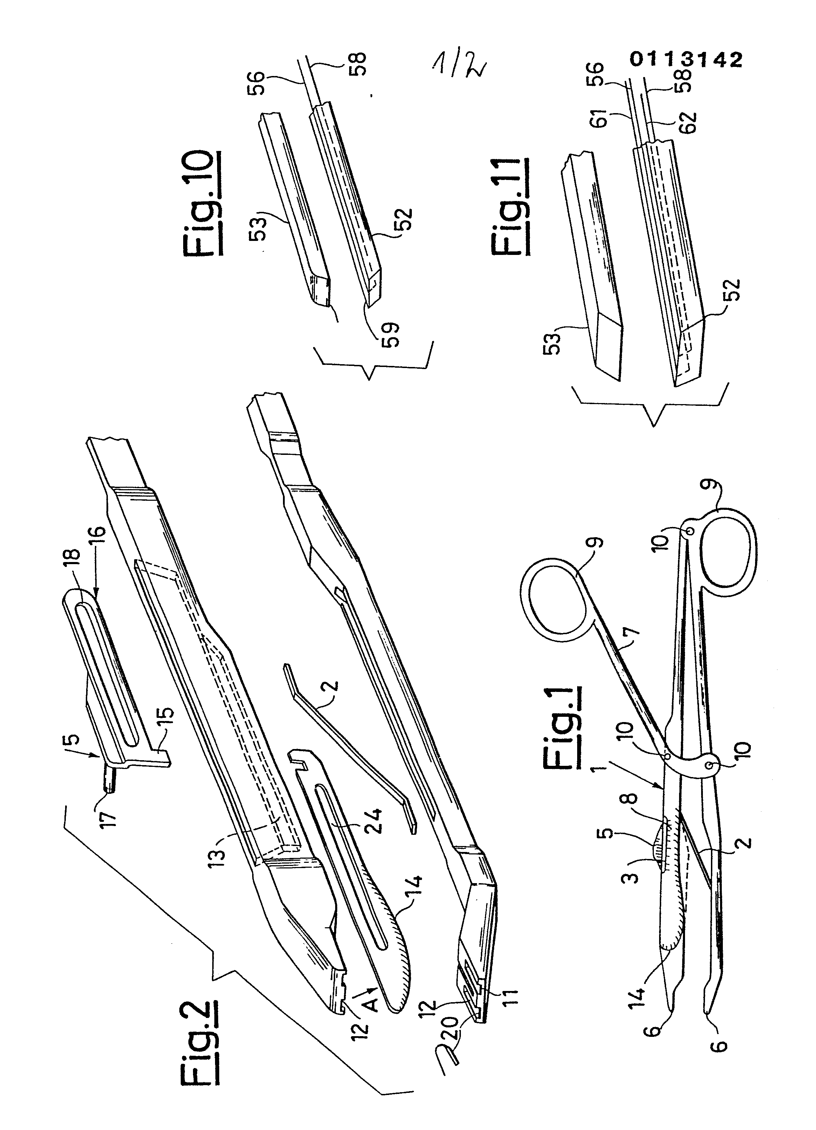

In the embodiment shown in Figures 1 to 5, the reference numeral 1 indicates a metal element surface-treated in such a manner as not to reflect light, and to be also easily sterilisable by normal industrial processes, and the reference numeral 2 indicates a flat spring which enables the forceps to open on termination of the required cycle of operations.

The reference numeral 3 indicates an inner compartment to house the sliding and cutting system, and provided according to a characteristic of the invention with transversely spaced-apart axial guides 4 with a locking notch 4a, for the insertion of the bistoury 14 which is operated by the device 5.

The reference numeral 6 indicates the forceps prongs, which can be at an appropriate angle to the longitudinal main axis of the forceps, and the reference numeral 7 indicates a system which enables the forceps to be closed to any required degree by means of a double lever linkage, the reference numeral 8 indicating a graduated scale which constantly displays the position of the cutting blade.

The overall forceps pivot on the pivots 10 when operated by the rings 9 (see Figures 1-3 in particular).

Each of the two forceps prongs 6 is provided with ends comprising steps 11 and grooves 12 for housing the suture clips 20 of substantially U configuration, and the reference numeral 13 indicates the axial channel which enables the blade of the bistoury 14, controlled by the device 5 comprising the appendix 15 of the rod 16, which is provided with a knob 17, to slide in the slot 18 in order to effect its cutting function (see Figures 2-4).

The surgical instrument according to the invention operates as follows.

When binding and cutting vessels and/or tissues, the surgeon utilises the forceps 1 by acting on their rings 9 to consequently immediately open the forceps. Then after inserting the appropriate suture clips 20 into the grooves 12, facilitated by the steps 11, the relevant vessels and/or tissues are progressively obliterated simultaneously upstream and downstream.

When obliteration has been effected, the operator manipulates the knob 17 in order to gradually make the cut in the tissue disposed between the two clips 20, and on termination of this cut, whether gradual and/or clean, he easily and rapidly returns the blade of the bistoury 14 manually by moving it into a passive and advantageously shielded position. After these operations, the forceps can be cyclically reopened by again manoeuvring their rings 9.

A further specific characteristic of the invention is that the bistoury 14, which is constructed of special steel and surface-treated by known specific heat treatment, is ground at 25 both on its lateral peripheral surface and on its point but to varying extents, so as to allow a sharpening variability, and a variability in the geometry of the cut itself.

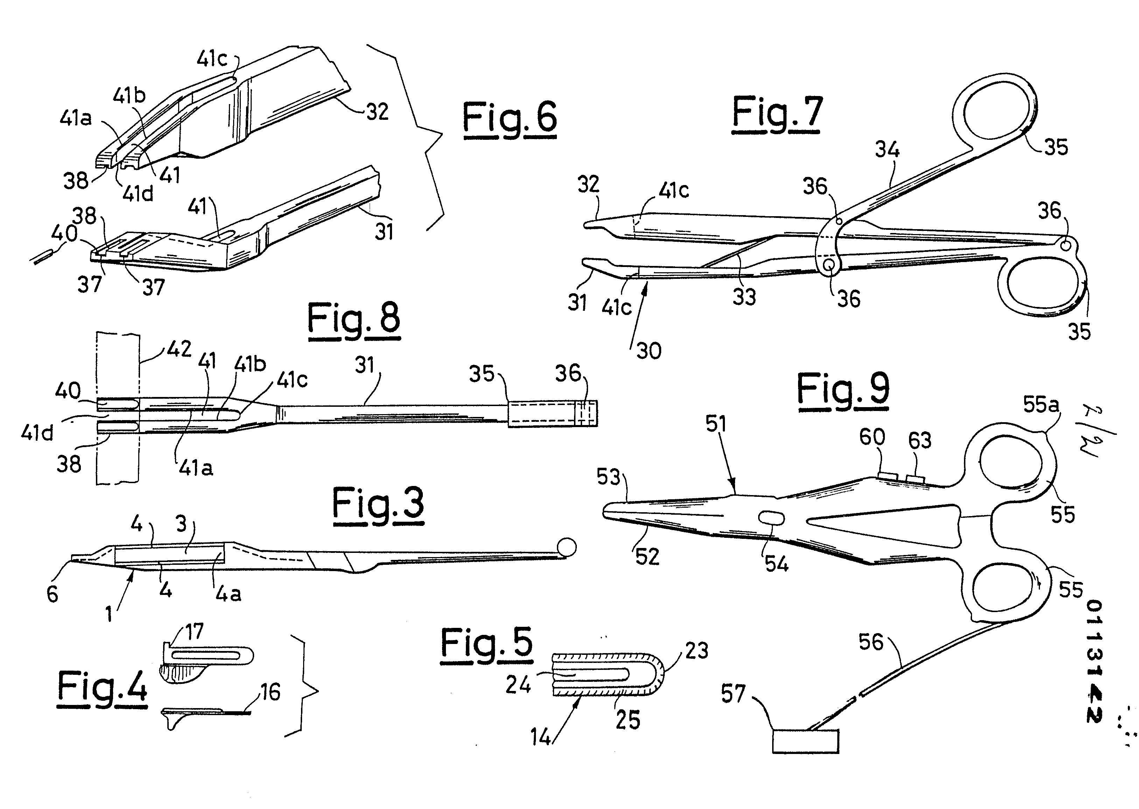

Said frontal point of the bistoury, indicated by 23, is of curved connection profile in order to allow gradual penetration in separating the edges of the tissue, so preventing traumatic incisions in the vessels or tissues concerned. The reference numeral 24 indicates a universal slotted connector in the bistoury 14 for grips of various configuration (see Figure 5).

Said structure of the bistoury 14, together with its different degrees of grinding, means that an instrument is provided for the surgical operation which is able not only to produce an incision cut with a certain pressure, but is able to usefully act uniformly
and rectilinearly.

This latter valuable characteristic together with those deriving
from the aforesaid description proves the optimum functionality of
the instrument, its rational and uncomplicated construction, and
the easy and rapid replacement and interchangeability of its
components.

In the second embodiment shown in Figures 6 to 8, the reference
numeral 30 indicates overall the forceps of the type according to
the invention, constructed of metal or possibly of hardened plastics,
which in both cases presents a non-reflecting surface to light
during the surgical operation, and also allows easy sterilisation.
Said forceps can either be of the normal scissors profile as already
illustrated with reference to the first embodiment, or can be
of the already mentioned specific forceps configuration. With
reference to the first embodiment, the reference numerals 31 and
32 indicate the respective prongs which are kept open during the
operation of the forceps by the flat spring 33, and the reference
numeral 34 indicates the operating lever for opening and closing
said instrument by the operator manoeuvring the two rings 35,
one of these, which are corresponding and similar, being incorporated
directly into the end of the lower prong 31, the three thus configured
elements being pivoted at the respective points 36 (see Figure 7).
Both the prongs 31, 32 of the forceps 30 are provided with ends
comprising steps 37 determined by the grooves 38, which are
arranged to contain the suture clips 40 of substantially U geometrical
configuration.

According to the specific inventive characteristic, the aforesaid end sectors of each of said prongs are provided with a slot 41 of limited axial length but passing completely through in a transverse direction, and arranged to define a pair of parallel spaced-apart walls 41a, 41b, which are raidally bounded by the internal joining portion 41c, said slot being open at its opposite end 41d. This configuration thus gives excellent visibility, being therefore effectively useful in obliterative operation on the tissue or vessel 42 by the surgeon.  This operation is easily executed by opening the forceps by the operator manipulating their rings 35, and then inserting the appropriate clips 40 into the grooves 38, they then being expelled to progressively clamp the requisite vessels and/or tissues simultaneously upstream or downstream of the surgical incision or cut to be made, this then being done separately by adding a further known forceps instrument. Because of the presence of said two slots 41, this clamping can be done with clips 40 positioned at a greater distance apart than is normal, and can be advantageously utilised in different and mutually orthogonal planes, a further new operational advantage being attained by providing the ends of the prongs 31, 32 with a suitable curved profile to allow surgical operation on the surfaces of organs which have a curved pattern of various profiles, or in cavities, this being all attained as heretofore described by a simple constructional arrangement of the forceps according to the invention.

With regard to the third embodiment illustrated in Figures 9-11, the description will be limited in terms of its parts only to those which are different from the two preceding embodiments, the reference numeral 51 indicating overall the forceps according to the invention.

Said forceps, constructed of non-conducting plastics material and thus insulating against the electrical effects described hereinafter, and also being non-reflecting and easily sterilisable, are formed in known manner from the two prongs 52, 53, which are pivoted on the pivot 54 and terminate in a ring-shaped end 55 comprising a protuberance 55a, for the insertion of the fingers for operation by the operator (see Figure 9).

According to the specific characteristic, in said prong, namely the lower prong 52, a suitable axial recess houses the suitably screened electric wire 56 which carries current from a high frequency generator 57 and returns it thereto through the underlying wire portion 58.

The respective ends of each prong comprise grooves for housing the known clips 59 of U configuration for effecting the necessary suture in consequence of the cut (see Figure 10).

The description demonstrates the ease of operation and excellent surgical intervention of the forceps by virtue of the elimination of the metal bistoury, resulting in a clean cut free from any tissue fringes of which the cells would negatively interfere with the instrument, this all being attained by virtue of the electric wire 56, which is made operative conventionally by incandescence, by operating the forceps activation pushbutton 60.

As shown in Figure 11, a further improvement is the elimination of the clips 59, which could always be difficult to operate and are bulky. In this useful simplification, the two effects, ie cutting and coagulation, are determined by suitably varying the current intensity and power, this latter effect being attained by the further electric wire 61, with its current return cable to the generator 57, all controlled by a further activation pushbutton 63 positioned on the upper edge of the forceps 51 adjacent to the said pushbutton 60, and thus both easy and rapid to operate by the operator (see Figure 11).

This embodiment therefore covers a partially electric design (suture clips 59 retained) followed by a totally electric design (clips 59 absent), but both utilising electro-surgical techniques able to positively satisfy the requirements of modern surgery such as microcoagulation, precise cuts with or without coagulation, and cutting or coagulation in aqueous solutions, a further advantage being the possibility of connecting the electro-bistoury to equipment by simple connectors.

By way of example, for an operation involving a pure cut, the instrument requires a power availability of from 0 to 300 W, and for coagulation purposes a pulsating high frequency current of between 20 and 50 kHz. A further advantage is that the said prongs 52, 53, which are suitably profiled with variable geometry, enable the forceps 51 to operate on organs with surfaces of any curvature, and in cavities.

Within the principle of the invention, modifications can be made to the described embodiments in order to further increase their functionality, without leaving the scope of the present invention as protected by the following claims.

## PATENT CLAIMS

1.　A medical-surgical instrument characterised by being structurally and substantially constituted by forceps which, at the ends of their respective prongs, incorporate adjacent means for effecting the suture respectively and simultaneously upstream and downstream of the relevant sector of a vessel or tissue in which a gradual operation involving incision and cutting is to be carried out by further means incorporated in said forceps, and performed by the surgeon.

2.　A medical-surgical instrument as claimed in claim 1, characterised in that the means for effecting the suture are substantially U-shaped clips contained in suitable side-by-side seats defined in the facing ends of the two prongs of the forceps; the means for executing the incision and cut being constituted by a bistoury slidably contained in a cavity in one prong in order to operationally act on said relevant sector between the two clips.

3.　A medical-surgical instrument as claimed in claim 1, characterised in that the bistoury slides under manual guidance between a withdrawn or retracted position and an extracted or operating position, means for displaying the operating position of the bistoury being associated with the graduated forceps closure device.

4.　A medical-surgical instrument as claimed in claim 2, characterised in that in the end of each prong between the seats incorporating the suture clips there extends a slot of limited extension but passing completely through transversely, and provided to correspond with the position in which the bistoury is located when in its extracted operating position.

5.      A medical-surgical instrument as claimed in claim 1, characterised in that the prongs have end curved profiles which enable the suture clips to be positioned at different distances apart and in order to operate on tissues or vessels which can be mutually orthogonal, and on organ surfaces of varying curvatures and cavities.

6.      A medical-surgical instrument as claimed in claim 1, characterised in that the means for making the incision and cut are constituted by an electric current-conducting wire arranged to form an electro-bistoury.

7.      A medical-surgical instrument as claimed in claims 1 and 6, characterised in that the suture operation is also effected by the incandescence of an electric wire, of which the current intensity and power vary according to the cutting operation.

8.      A medical-surgical instrument as claimed in claims 6 and 7, characterised in that the operation of said two electric wires, which are suitably screened and comprise a return line to a power generator, is activated by a pushbutton.

Fig.2

Fig.10

Fig.1

Fig.11

0113142

**Fig.7**

**Fig.6**

**Fig.8**

**Fig.9**

**Fig.3**

**Fig.5**

**Fig.4**